# EUROPÄISCHE PATENTSCHRIFT

(11) **EP 1 486 186 B1**
(45) Veröffentlichungstag und Bekanntmachung des Hinweises auf die Patenterteilung: **05.09.2007**
(21) Anmeldenummer: 04013320.9
(22) Anmeldetag: 05.06.2004
(51) Int. Cl.: A61F 11/00

(54) **Gerät zur Behandlung von Tinnitus**
Apparatus for treating tinnitus
Dispositif de traitement du tinnitus

(30) Priorität: 12.06.2003 DE 10326522
(43) Veröffentlichungstag der Anmeldung: 15.12.2004
(73) Patentinhaber: Strasser, Johanna, 92521 Schwarzenfeld (DE)
(72) Erfinder: Lenke, Michael, 6342 Baar (CH)
(74) Vertreter: Pausch, Thomas Ernst

(56) Entgegenhaltungen:
- EP-A- 0 472 490
- WO-A-01/08617
- WO-A-02/087698
- DE-A- 10 049 068
- GB-A- 2 366 734
- SE-A- 9 803 233
- US-A- 3 841 321

## Beschreibung

Die Erfindung betrifft ein Behandlungsgerät zur Reduzierung von Ohrgeräuschen, insbesondere zur Beseitigung von Tinnitus, nach dem Oberbegriff des Anspruches 1.

Ein solches Behandlungsgerät ist aus SE-A-9 803 233 bekannt geworden. Bei diesem werden die den Tinnitus verursachenden Ohrgeräusche unter Verwendung justierbarer Lichtwellen dadurch vermindert, dass diese mit defekten Hörnervsignalen interferieren.

Aus der WO 97/10794 ist ein Vibrationshandgerät bekannt geworden, dessen Vibrationskopf dergestalt ausgeformt ist, dass er auf den Eingang des Gehörganges aufgesetzt werden und diesen durch Vibration stimulieren kann. Dieses bekannte Massagegerät ist weder bestimmt noch geeignet zur wirksamen Behandlung von unter der Tinnitus-Krankheit leidenden Personen.

Aufgabe der vorliegenden Erfindung ist es daher, ein zur Reduzierung von Ohrgeräuschen geeignetes, kostengünstig herstellbares, handliches kompakt bauendes Behandlungsgerät vorzuschlagen, welches von einer betroffenen Person selbst benutzt werden kann, und welches gegenüber den bekannten Behandlungsgeräten deutlich wirksamer zur Behandlung von Tinnitus ist.

Diese Aufgabe wird gelöst durch die Merkmale des Anspruchs 1.

Dabei wird erstmals eine Vorrichtung zur Reduzierung von Ohrgeräuschen, insbesondere zur Beseitigung von Tinnitus vorgeschlagen, bei dem zugleich eine Bestrahlung und eine Vibrations-Massage zumindest eines Abschnitts des sich von der Ohrmuschel bis zum Trommelfell erstreckenden Gehörganges vorgesehen ist. Dabei soll von wenigstens einer Leuchtdiode erzeugte, vorzugsweise monochromatische, Strahlung vermittels eines Lichtleiters dem zu bestrahlenden, den Gehörgang begrenzenden Gewebe zugeführt und dort, vorzugsweise diffus verteilt, abgegeben werden. Wobei zugleich von einer Vibrationsquelle erzeugte Vibrationen dem Gehörgang in dessen vorderen, der Ohrmuschel zugewandten Eingangsbereich aufgeprägt werden.

Durch die Verwendung von Leuchtdioden, kurz LEDs als Strahlungsquelle steht eine für das menschliche Auge weitestgehend ungefährliche Lichtquelle zur Verfügung. Auf diese Weise sind die von Lasertherapien bekannten Nachteile der Verletzungsgefahr für die Augen beim Hantieren mit dem Behandlungsgerät erheblich minimiert. Zudem bieten Leuchtdioden den weiteren Vorteil, daß diese zwar auch wie Laser monochromatisches Licht zur Verfügung stellen können, deren Licht bzw. Strahlung schwingt jedoch nicht absolut parallel und ist daher sozusagen sanfter. Mit anderen Worten: Die Strahlung von Leuchtdioden ist zur Bestrahlung des menschlichen Gewebes im Gehörgang und auch insbesondere hinter dem Trommelfell wesentlich besser geeignet.

Die von LEDs erzeugten, vorzugsweise monochromatischen, Photonen werden aufgrund deren guter biologischer Verträglichkeit auch Biophotonen genannt. Die Biophotonen helfen, den überbeanspruchten menschlichen Organismus und damit ein überreiztes Gehör ins Gleichgewicht zu bringen und können damit Gesundheitsstörungen, wie beispielsweise Ohrgeräusche oder gar Tinnitus lindern.

Wenngleich die zugrundeliegenden Effekte wissenschaftlich noch nicht erforscht sind, scheint es so zu sein, daß die von den Leuchtdioden erzeugten Photonen einen zellulären Energienotstand beheben können. Die von den Leuchtdioden bzw. von der Leuchtdiode erzeugte Lichtenergie bzw. Strahlung regt die in einer Zelle enthaltenen Mitochondrien an und produziert dabei in der angeregten Zelle den Zellkraftstoff Adenosintriphosphat (ATP). Diese neu produzierte, von der angeregten Zelle abgegebene molekulare Zellenergie belebt den betreffenden Gewebebereich und es kann zu einer signifikanten Verbesserung der Gesundheitsstörung kommen, es ist also eine Reduzierung der Ohrgeräusche bis hin zu einer Beseitigung eines Tinnitus feststellbar.

Dieser positive Effekt auf das den Gehörgang begrenzende Gewebe durch die vom Lichtleiter in den Gehörgang eingeprägte Strahlung wird durch die dem Gehörgang ebenfalls aufgeprägte Vibrationen zusätzlich verstärkt. Dabei scheint es so zu sein, daß die im Bereich des Gehöreingangs aufgeprägten Vibrationen sich auf den dortigen Knorpel und den sich daran anschließenden Knochenabschnitt des menschlichen Schädels übertragen lassen. Die auf diese Weise dem den Gehörgang umschließenden Knochenabschnitt eingeprägten Schwingungen bzw. Vibrationen pflanzen sich bis zur Paukenhöhle und von dort weiter bis zum inneren Gehörgang fort. Dabei wird angenommen, daß die Knochenschirmung des Gehömervs somit ebenfalls von diesen Vibrationen erfaßt wird und damit eine Auslöschung der störenden Ohrgeräusche oder des Tinnitus bzw. eine Art Neunivellierung des Gehörs oder in gewissem Sinne ein Reset des Nullniveaus der gestörten Frequenzen stattfinden kann. Hierbei scheinen sich die positiven Wirkungen der Strahlung mit den positiven Effekten der Vibration zu überlagern und gegenseitig zu unterstützen.

Obwohl diese Effekte noch nicht erforscht sind, konnten gleichwohl bereits erste Linderungserfolge bei Selbstversuchen mit Testpersonen festgestellt werden. Inwieweit es hier eine Rolle spielt, daß die Gehör-Schnecke, das eigentliche Hörorgan, aus einem knöchernen und einem häutigen Teil besteht und der knöcherne Teil die Fasern des Hömervs enthält, ist noch nicht abschließend geklärt. Jedenfalls scheint es wohl so zu sein, daß sich die am Eingang des Gehörganges eingeprägten mechanischen Vibrationen sich über die Knochenstruktur bis zum inneren Gehörgang bzw. bis zur Schnecke fortpflanzen und dort positive Effekte auslösen, die letztendlich zumindest zu einer Linderung von Ohrgeräuschen wenn nicht sogar bis zu einer vollständigen Heilung eines Tinnitus führen können. Dabei ist anzunehmen, daß die knöchernen Teile des Hörorgans positiv von den Vibrationen und die häutigen Teile des Hörorgans positiv von der Strahlung stimuliert werden, so daß letztendlich in Zusammenwirkung dieser Anregungen die Linderung von Ohrgeräuschen und sogar eine Beseitigung von Tinnitus verzeichnet werden kann.

Mit der Erfindung wird ein Behandlungsgerät zur Reduzierung von Ohrgeräuschen, insbesondere zur Beseitigung von Tinnitus, vorgeschlagen, bei dem mittels Bestrahlung und Massage zumindest eines Abschnitts des sich von der Ohrmuschel bis zum Trommelfell erstreckenden Gehörganges, eine Linderung der Ohrgeräusche bzw. Heilung des Tinnitus erzielbar wird. Hierbei weist das Behandlungsgerät ein Gehäuse auf. In dem Gehäuse ist eine Energiequelle angeordnet, die mit einem Stromkreis und ggf. einer Schaltung zur Regelung der Energiezufuhr mit einer Lichtquelle verbunden ist. Dabei ist als Lichtquelle wenigstens eine Leuchtdiode vorgesehen, wobei das von der Leuchtdiode imitierte Licht von einem Lichtleiter aufnehmbar und einem zu bestrahlenden, den Gehörgang begrenzenden Gewebe zuführbar ist. Ferner ist im Gehäuse eine Vibrationsquelle angeordnet, die mit der Energiequelle über einen weiteren Stromkreis und ggf. eine Schaltung zur Regelung der Energiezufuhr verbunden ist. Hierbei sind die von der Vibrationsquelle erzeugten Vibrationen mit einem Übertragungselement dem Gehörgang in dessen vorderen, der Ohrmuschel zugewandten Eingangsbereich aufprägbar.

Das erfindungsgemäße Bestrahlungsgerät ist klein, handlich und liegt dank seiner ergonomischen Form gut in der Hand. Es verfügt über selbsterklärende Bedienungselemente und ist aufgrund der ausgefeilten, zum Teil auf konkrete Behandlungsmuster abgestimmten Elektronik einfach in der Anwendung und kann damit von jedermann bedient werden. Ferner ist es preiswert in der Anschaffung und somit für jedermann erschwinglich.

Vorteilhafte Weiterbildungen und Aspekte des erfindungsgemäßen Behandlungsgeräts ergeben sich aus den jeweiligen Unteransprüchen.

So ist bei einer bevorzugten Verwendungsform des Behandlungsgeräts die Intensität und/oder die Dauer der Bestrahlung regelbar. Diese Regelung kann beispielsweise vermittels entsprechend konfigurierter Schaltungen, über eine, vorzugsweise programmierbare, Elektronik, über Bedienungselemente oder dergleichen erfolgen. Damit können in vorteilhafter Weise die mittels der Photonen von der Leuchtdiode dem Gewebe zugeführten Energien auf den jeweiligen Bedarf abgestimmt werden. Damit wird einerseits eine überhöhte Energiezufuhr vermieden und andererseits ist ausgeschlossen, daß eine Behandlung beendet wird, bevor dem Gewebe bzw. den Mitochondrien ausreichend neue Energien zugeführt worden sind

Weiterhin ist bekannt, daß Heilungsprozesse häufig dem Placeboeffekt unterliegen. Demzufolge wird bei einer weiter bevorzugten Ausgestaltung des Verfahrens die Strahlung moduliert, insbesondere gepulst. Eine gepulste Strahlung impliziert dem Menschen unterbewußt eine höhere Energiedichte und damit eine stärkere Heilungswirkung. Folglich empfindet ein Mensch, der die erfindungsgemäße Vorrichtung benutzt bei einer gepulsten Strahlung eine bessere bzw. gezieltere Heilung. Darüber hinaus kann die Behandlungsdauer mit einer gepulsten Strahlung auf größere Zeiträume ausgedehnt werden, als dies bei einer Dauerbestrahlung möglich ist, ohne punktuelle Überdosen in den äußeren, der Strahlung direkt zugewandten Gewebeschichten des Gehörganges oder des hinter dem Trommelfell liegenden Innenohres befürchten zu müssen, da die pro Impuls eingebrachte Energiemenge während der nachfolgenden Pause vollständig von den dortigen unteren Gewebeschichten aufgenommen werden kann.

In einer weiter bevorzugten Verwendungsform des Behandlungsgeräts wird eine Strahlung im Wellenlängenbereich von 610 nm bis 660 nm, bevorzugt von 630 nm bis 640 nm gewählt. In ersten Versuchen hat sich herausgestellt, daß insbesondere Licht im Wellenlängenbereich von 610 nm bis 660 nm von Leuchtdioden eine besonders ausgeprägte, ohrgeräuschauslöschende Wirkung hat. In manchen Fällen waren deutliche Besserungen bereits nach Bestrahlungen mit gleichzeitiger Gabe von Vibrationen von knapp zwei Minuten Dauer feststellbar.

Die von ultrahellen Leuchtdioden abgegebene Lichtstärke beträgt in etwa ab 10.000 Mikrocandela bis 20.000 Mikrocandela. Zur Erzielung einer raschen Besserung kann man das Behandlungsgerät bei Lichtstärken von etwa 15.000 bis 25.000 Mikrocandela betreiben.

Gemäß einer weiter bevorzugten Ausführungsform des Behandlungsgeräts ist vorgesehen, daß die Intensität und/oder die Dauer der Vibrationen geregelt wird bzw. regelbar ist. Hierbei sollen in einer weiter bevorzugten Ausführungsform die Vibrationen auf 2.000 bis 5.000 Schwingungen pro Minute, vorzugsweise auf maximal 4.500 Schwingungen pro Minute und besonders bevorzugt auf maximal 3.000 Schwingungen pro Minute eingeregelt werden können.

Als Vibrationsquelle kann beispielsweise ein aus dem Telekommunikationsbereich für die Herstellung von Handys bekannter Vibrationsmotor Verwendung finden. Derlei Vibrationsmotoren zeichnen sich durch deren kompakte, robuste Bauweise und extrem hohe Standzeiten aus. Bei Behandlungszeiten von beispielsweise zwei bis fünf Minuten täglich vormittags und abends lassen sich damit problemlos Lebensdauerzeiten von über drei Jahren bei täglicher Benutzung erzielen. Wenn man davon ausgeht, daß eine zu behandelnde Person bereits nach wenigen Tagen spätestens nach einigen Wochen frei von störenden Ohrgeräuschen ist, können damit eine Vielzahl von unter Ohrgeräuschen leidenden Personen bereits mit einem einzigen Gerät Linderung erfahren. Damit verstärkt sich noch der kostengünstige Effekt des erfindungsgemäßen Behandlungsgeräts.

In einer weiter bevorzugten Ausführungsform ist vorgesehen, daß die Vibrationen gepulst werden. Damit lassen sich die bereits vorstehend zur Bestrahlung diskutierten positiven Effekte auch auf die Einprägung der Vibration übertragen.

In einer bevorzugten Ausführungsform des Behandlungsgeräts weist dieses einen mit einem Überzug, einer Schutzhülle oder einem Schutzfilter überziehbaren Lichtwellenleiter auf. Indem der Abschnitt des Lichtwellenleiters, der in den Gehörgang gesteckt wird, mit einer Schutzhülle überzogen werden kann, ist ein Gerät von mehreren Personen benutzbar, ohne dabei die erforderliche Hygiene vernachlässigen zu müssen. Jede Person, die das Gerät benutzen möchte, setzt vor der Benutzung eine neue Schutzhülle auf das vordere Ende des Lichtwellenleiters, so daß eine optimale Hygiene gewährleistet.

Gemäß einer weiter bevorzugten Ausführungsform ist der Lichtwellenleiter am lichtemittierenden Ende gewölbt ausgebildet oder halbkugelartig abgerundet. Damit wird eine diffuse Verteilung des austretenden Lichts erreicht. Bei einer diffusen Verteilung des austretenden Lichts wird dieses flächig gestreut. Damit ist die Gefahr eine punktuellen Überbestrahlung ausgeschlossen. Die pro Gewebefläche eingebrachte Strahlungsenergie bzw. -dichte bleibt somit gering und ist letztlich auf ein akzeptables Maß begrenzbar. Auf diese Weise, können auch bei Bedarf relativ hohe Energien pro Zeit dem den Gehörgang begrenzenden Gewebe wie auch dem hinter dem Trommelfell liegenden Gewebe zugeführt werden, ohne dabei schädigende Spitzen zu riskieren.

Die abgerundete Form des emittierenden Endes des Lichtwellenleiters stellt dabei sicher, daß der Gehörgang nicht verletzt werden kann, auch nicht versehentlich. Zudem dient sie einerseits dazu, Licht rundherum möglichst breitflächig bzw. raumgreifend an das umgebende Gewebe abzugeben als auch um Licht nach vorne gerichtet beispielsweise bis zum Trommelfell und durch dieses hindurch vordringen zu lassen. Mit dem erfindungsgemäßen Behandlungsgerät kann somit bereits nach zwei Minuten Bestrahlung und Einprägung von Vibrationen in den Gehörgang ein spürbares Abklingen von Ohrgeräuschen und zum Teil sogar eine vollständige, "spontane" Heilung von Tinnitus festgestellt werden.

Gemäß einer weiter bevorzugten Ausführungsform ist vorgesehen, daß die Lichtquelle von einer ultrahellen roten LED gebildet wird. Damit sind die bereits vorstehend diskutierten Lichtstärken von 10.000 µcd (Mikrocandela) bis 20.000 µcd (Mikrocandela) problemlos erzielbar und man bewegt sich damit bereits in einer Leistungsdichte, die ansonsten nur mit wesentlich teureren und gefährlicheren Softlasern erzeugbar ist.

Wie bereits vorstehend zum Teil angesprochen, ist in einer besonders bevorzugten Ausführungsform des Behandlungsgeräts vorgesehen, daß die Vibrationsquelle von einem Vibrationsmotor gebildet ist, der zwischen 2.000 bis 5.000 Schwingungen pro Minute erzeugen kann. Hierfür sind aufgrund der hohen Lebensdauer und Strapazierfähigkeit sowie wegen der kompakten, extrem kleinen Bauform als auch wegen der äußerst geringen Leistungsaufnahme die Vibrationsmotoren von Handys besonders gut geeignet. Zudem können diese Vibrationsmotoren bereits ausreichend starke Vibrationen erzeugen, die dann auch vom Eingang des Gehörganges über den Schädelknochen bis hin zu den knöchernen Teilen der Schnecke im inneren Gehörgang weitergeleitet werden können.

Hierfür ist in einer besonders bevorzugten Ausführungsform vorgesehen, daß die Vibrationsquelle mit einem Übertragungselement gekoppelt ist, das ringförmig ausgebildet ist. Das ringförmige Übertragungselement soll dabei bevorzugt eine hautfreundliche Oberfläche aufweisen. Ferner soll der Lichtwellenleiter durch das ringförmige Übertragungselement hindurch geführt sein. Auf diese Weise kann mit dem ringförmigen Übertragungselement der Eingangsbereich des Gehörganges vollständig abgedeckt und zugleich der Lichtwellenleiter bzw. dessen in den Gehörgang ragender Abschnitt in den Gehörgang eingeführt werden. Damit ist sichergestellt, daß die in den Gehörgang gesandte Strahlung nicht versehentlich zwischen dem Gehöreingang und dem Gerät austreten kann. Zugleich sorgt die hautfreundliche Oberfläche des dem Gehörgang zugewandten Abschnittes des Übertragungselements dafür, daß ein besonders gutes Anschmiegen oder Anpassen des Übertragungselements an den Gehöreingang sichergestellt ist. Zugleich kann beispielsweise mit einer Beschichtung aus einem weichen Gummi oder dergleichen sichergestellt werden, daß man ein angenehmes Empfinden bei der Benutzung wahrnimmt. Damit wird die Akzeptanz des Behandlungsgerätes bei der Benutzung erhöht. Die ringförmige Ausgestaltung des Übertragungselements ähnelt am ehesten der Kontur des Gehörgangeinganges. Gleichwohl können auch andere Übertragungselemente vorgesehen werden, die beispielsweise speziell dem jeweiligen Gehöreingang angepaßt werden. Je besser das Übertragungselement sich dem Gehöreingang anschmiegt, um so besser können die Vibrationen vom Übertragungselement auf diesen Bereich des Gehörganges übertragen und damit letztendlich auf die betroffenen Abschnitte des Schädelknochens eingeprägt werden.

Es sind auch Alternativen vorstellbar, bei denen der Lichtwellenleiter bzw. dessen durch das Übertragungselement hindurch ragender Abschnitt, der in den Gehörgang eingeführt wird, und das Übertragungselement mechanisch miteinander als eine Einheit ausgeführt bzw. verbunden sind. Dann schwingt das die Strahlung emittierende Ende des Lichtleiters mit der Vibration des Übertragungselements mit, was sich aufgrund der geringen Schwingungslänge bzw. des geringen Vibrationshubes nicht weiter negativ auswirkt. Hier wird der letztendlich wohl der kostengünstigsten Bauform der Vorzug zu geben sein.

In verschiedenen Tests konnte festgestellt werden, daß bereits ein Hub von weniger als 1 mm oder sogar weniger als 0,5 mm ausreicht, um eine entsprechende Vibrationsenergie über den Gehöreingang auf das dahinter liegende Gehör bzw. die dahinter liegende Knochenstruktur zu übertragen. Dabei kommt es letztendlich vermutlich weniger auf die übertragene Kraft pro Hub bzw. pro Schwingung sondern mehr auf die Kombination aus Vibration plus Bestrahlung mit Licht an.

In einer weiter bevorzugten Verwendungsform des Behandlungsgeräts wird die Abgabe der Lichtstrahlung durch die Erzeugung eines korrespondierenden Tonsignals vermittels eines Schallgebers bzw. Lautsprechers oder dergleichen hörbar gemacht. Die akustische Vemehmbarkeit der Lichtstrahlung bzw. deren hörbares Pulsieren bietet dem Benutzer ein Feedback. d.h. eine Rückmeldung zur laufenden Behandlung und führt damit zu einer höheren Akzeptanz. Ein Benutzer erhält damit das Gefühl, er beherrsche das Behandlungsgerät bzw. den Prozeß, da er dessen Funktionieren oder Nicht-Funktionieren hören kann. Das sonst häufig aus der Praxis bekannte, beklemmende Gefühl, einer Behandlungsmethode hilflos ausgeliefert zu sein, wird somit in vorteilhafter Weise vermieden, was sich wiederum positiv auf das Empfinden der betroffenen Person auswirkt. Indem die heilende bzw. positive Wirkung mit der Benutzung des Geräts bzw. des Verfahrens hörbar wird, führt dies zu positiven Nebeneffekten im Unterbewußtsein, die den Heilungserfolg zusätzlich stimulieren. Damit ist es erstmals möglich, ungewollte Ohrgeräusche rasch zum Abklingen zu bringen oder sogar in manchen Fällen einen Tinnitus zu heilen. Hierbei kann in vorteilhafter Weise der Effekt genutzt werden, daß gezielt abgegebene Schallwellen auch ein überreiztes Gehör positiv beeinflussen, es sozusagen neu einjustieren oder neu nivellieren können.

In Kombination mit der vorstehend diskutierten Regelung der Strahlung können damit die unterschiedlichsten Behandlungsprogramme vorgegeben werden. So kann ein Behandlungsprogramm beispielsweise vorsehen, daß zwei Minuten lang Licht im roten Spektrum zusammen mit Geräuschen abgegeben wird. Daran anschließend können drei Minuten lang Licht im roten Spektrum mit Vibrationen die im Gehörgang aufgeprägt werden, wobei die Vibrationen mit 2.000 Umdrehungen pro Minute beginnen und sich bis auf 4.500 Schwingungen pro Minute kontinuierlich steigern. Wie erste Selbstversuche mit Testpersonen aus dem Entwicklungsteam zeigten, kann dabei mit Erfolgs- bzw. Linderungsquoten von 50 % oder mehr gerechnet werden.

Gemäß einer bevorzugten Verwendungsform des Behandlungsgerät wird das zu bestrahlende Gewebe der unterstützenden Wirkung eines Magnetfeldes ausgesetzt. Das Magnetfeld kann beispielsweise von einem Permanentmagneten erzeugt werden. Durch eine bei der Bestrahlung gleichzeitig stattfindende Magnetfeld-Stimulation wird eine Erhöhung der Gesamtwirkung erzielt.

Gemäß einer weiter bevorzugten Ausführungsform ist vorgesehen, daß das Gehäuse wenigstens ein Bedienelement, beispielsweise einen Regelknopf, einen Taster, einen Touch-Screen, einen Multifunktionsschalter oder dergleichen aufweist, vermittels dem die Intensität und/oder die Dauer und/oder die Frequenz der Strahlungen wie der Vibrationen sowie gegebenenfalls auch die weiteren Funktionen regelbar einstellbar sind, wobei vorzugsweise vorprogrammierte Steuerprogramme abrufbar sein sollen Damit wird die Handhabbarkeit des erfindungsgemäßen Behandlungsgeräts für die betroffene Person sehr einfach und es sind zugleich Fehlbedienungen ausgeschlossen.

Weitere Einzelheiten, Aspekte und Vorteile der vorstehend diskutierten Erfindung ergeben sich aus der nachfolgenden Beschreibung unter Bezugnahme auf die Zeichnung.

Es zeigen:
- Fig. 1: eine seitliche Ansicht einer beispielhaften Ausführungsform eines Geräts zur Reduzierung von Ohrgeräuschen, das in einen ausschnittsweise dargestellten, teilweise geschnittenen Gehörgang eingeführt ist;
- Fig. 2: das in Fig. 1 gezeigte Gerät für sich alleine;
- Fig. 3: eine seitliche Ansicht einer weiteren beispielhaften Ausführungsform des Geräts;
- Fig. 4: eine Frontansicht des in Fig. 3 gezeigten Geräts;
- Fig. 5: den Kopf des in Fig. 3 und 4 gezeigten Geräts mit Schutzhülle für den in den Gehörgang einzuführenden Abschnitt; und
- Fig. 6: den in Fig. 5 gezeigten Kopf des Geräts, wie er im Gehörgang eingeführt ist.

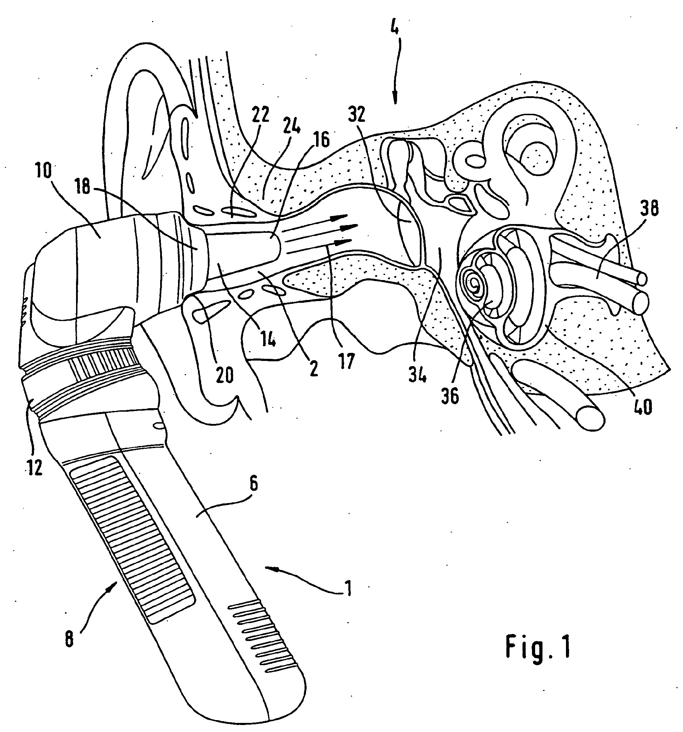

In Fig. 1 ist in schematischer Weise eine Ausführungsform eines erfindungsgemä-ßen Geräts 1 dargestellt.

In Fig. 1 ist eine Ausführungsform eines Behandlungsgeräts 1 zur Reduzierung von Ohrgeräuschen und insbesondere zur Beseitigung von Tinnitus gezeigt. Das Behandlungsgerät 1 ist zum Teil in den Gehörgang 2 eines im Schnitt dargestellten Gehörs 4 eingeführt. Das Behandlungsgerät 1 weist ein Gehäuse 6 auf, daß in ein als Handgriff ausgebildeten Abschnitt 8 und einen daran anschließenden Kopf 10 untergliedert ist. Der Kopf 10 und der Handgriff 8 sind über ein Gelenk 12 gelenkig miteinander verbunden. Das Gelenk 12 erleichtert die Handhabung des Geräts 1 und vermeidet ein Verkanten beim Einführen in den Gehörgang 2. Zudem entkoppelt das Gelenk 12 den Kopf 10 vom Handgriff 8, so daß sich etwaige Vibrationen vom Kopf 10 nicht auf den Handgriff 8 übertragen und umgekehrt bei der Anwendung eine unruhige Handhabung mit entsprechend wackeligem Halten des Handgriffs 8 keine Auswirkung auf die Stellung des Kopfes 10 zum Gehörgang 2 hat.

Der Kopf 10 enthält einen strahlungemittierenden Lichtleiter 14, dessen vorderer Abschnitt 16, der in der hier gezeigten Darstellung in den Gehörgang 2 eingeführt ist, dem Gewebe, das den Gehörgang 2 umgibt, eine mit den Pfeilen 17 symbolisierte Strahlung einprägt. Ein den Lichtleiter 14 umschließendes ringförmiges Übertragungselement 18 ist ebenfalls am Kopf 10 angeordnet und schließt den Gehörgang 2 bei Benutzung des Geräts 1 im Bereich des Gehöreingangs 20 weitestgehend dicht ab. Mit dem Übertragungselement 18 werden die Vibrationen auf die angrenzende Knorpelmasse 22 bzw. Knochenstruktur 24 übertragen.

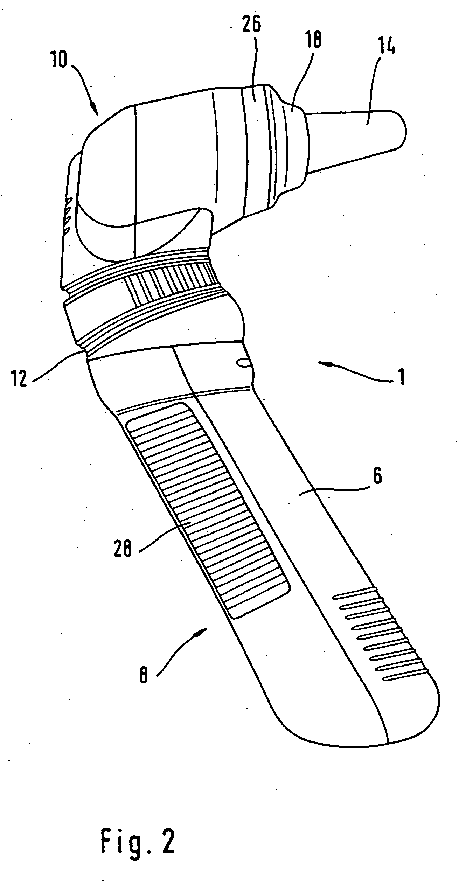

Das in Fig. 1 gezeigte Behandlungsgerät 1 ist in Fig. 2 in einer dreidimensionalen Ansicht für sich alleine veranschaulicht. Gleiche Elemente bzw. Bauteile werden zur Vermeidung von Wiederholungen mit den selben Bezugszeichen versehen. Der Kopf 10 des Geräts 1 enthält neben dem Lichtleiter 14 und dem Übertragungselement 18 in der hier dargestellten Ausführungsform ferner einen Magneten 26. Der Magnet 26 ist hierbei ringförmig ausgebildet und zwischen dem Gehäuserand des Kopfes 10 und dem Übertragungselement 18 angeordnet. Weiterhin umfaßt der Kopf 10 den dort nicht näher dargestellten integrierten Vibrationsmotor als Quelle für die Vibrationen.

Im Gehäuse 6 ist im als Handgriff 8 ausgebildeten Abschnitt ein Multifunktions-Bedienelement 28 vorhanden, das beispielsweise als Regelknopf, Taster, Touch-Screen oder wie hier der Einfachheit halber dargestellt als herkömmlicher Schiebeschalter ausgebildet sein kann. Damit läßt sich die Intensität und/oder die Dauer und/oder die Frequenz der Strahlungen wie auch der Vibrationen oder anderer Funktionen ein- bzw. ausschalten oder über entsprechende Steuerungen regeln bzw. vorprogrammierte Steuerprogramme abrufen. Im Gehäuse 6 sind zudem nicht näher dargestellte Miniatur-Lautsprecher integriert.

Im Gehäuse 6 ist ferner eine hier nicht dargestellte Energiequelle angeordnet, die mit einem Stromkreis und einer Schaltung zur Regelung der Energiezufuhr mit der Lichtquelle wie auch mit der Vibrationsquelle verbunden sein kann.

Das Übertragungselement 18 weist eine Beschichtung mit einem besonders weichen, hautverträglichen Material auf, um sich möglichst gut an das Ohr bzw. den Gehöreingang anzuschmiegen und die Vibrationen besonders gut zu übertragen. Gleichzeitig wird damit ein angenehmes Traggefühl bzw. Benutzungsgefühl erzielt.

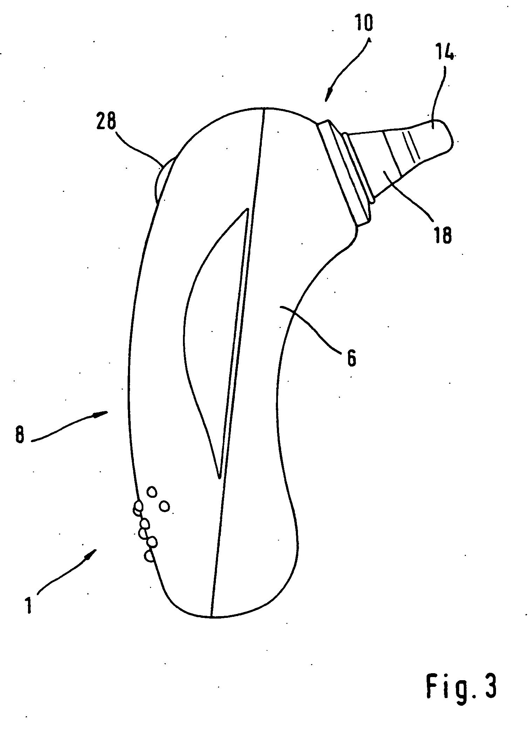

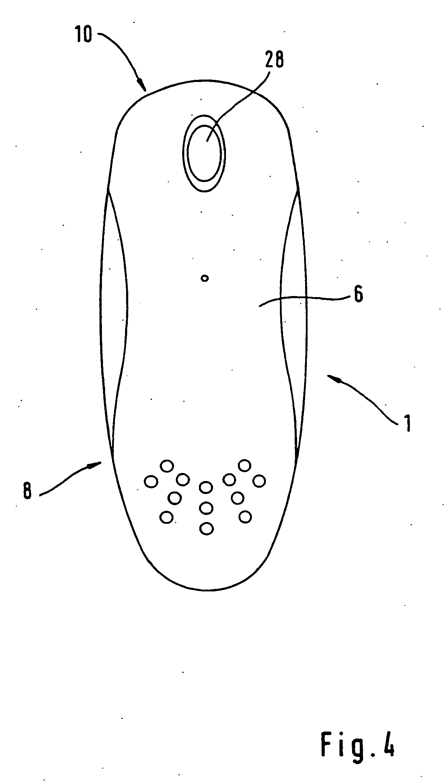

In Fig. 3 und 4 ist eine weitere Ausführungsform eines erfindungsgemäßen Behandlungsgeräts 1 in einer seitlichen Ansicht wie auch in einer Frontalansicht gezeigt. Diese Ausführungsform zeichnet sich durch eine besonders elegante Kontur aus und liegt zugleich äußerst gut in der Hand.

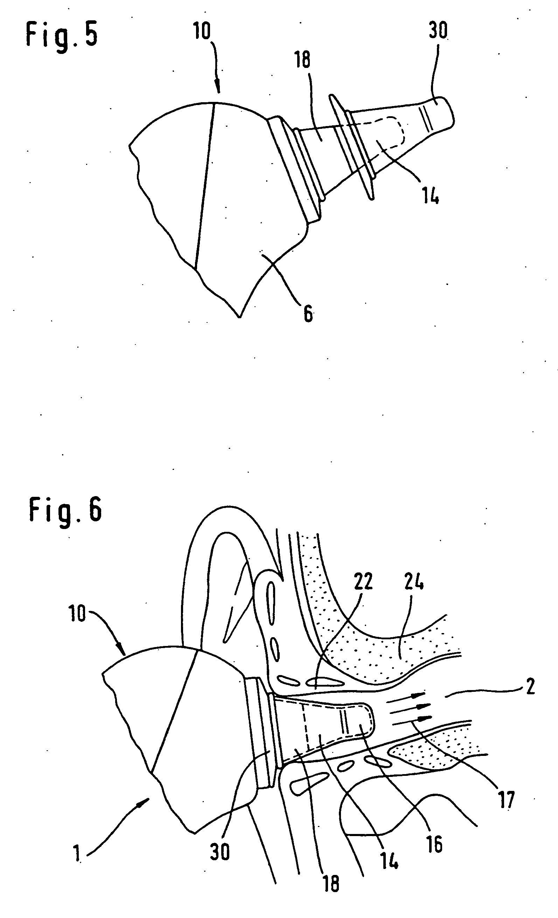

In Fig. 5 ist der Kopf 10 des Behandlungsgeräts 1 ausschnittsweise vergrößert dargestellt. Eine Schutzhülle bzw. ein auswechselbarer Überzug 30 kann dem die Strahlung 17 emittierenden Lichtleiter 14 übergezogen bzw. übergestülpt werden. Der Überzug 30 stellt eine ausreichende Einhaltung der Hygiene bei der Benutzung sicher. Damit können eine Vielzahl von Personen ein und das selbe Gerät benutzen. Es braucht hierfür lediglich immer wieder nach der Benutzung ein neuer Überzug 30 übergestülpt werden. Zudem kann der Überzug 30 zugleich ein auf bestimmte Wellenlängen abgestimmtes Filter oder eventuell eine zusätzliche Linse zur Fokussierung oder zur Streuung der Strahlung mit enthalten, so daß damit eine weitere Optimierung der Verteilung der Strahlung wie der bevorzugten Wellenlängen möglich wird.

In Fig. 6 ist der in Fig. 5 gezeigte Kopf 10 mit in den Gehörgang eingeführtem Lichtleiter 14 und darüber gestülptem Überzug 30 in Aktion gezeigt. Die emittierte Lichtstrahlung 17 reicht in den Gehörgang 2 hinein und gelangt bis zum Trommelfell 32, wie dies aus Fig. 1 besonders gut ersichtlich ist. Dabei kann die Strahlung 17 gegebenenfalls das Trommelfell 32 durchdringen und erreicht damit die dahinter liegenden Gehörteile, wie beispielsweise die Paukenhöhle 34, die Schnecke 36 und den inneren Gehörgang 38 sowie die weiteren Organe des menschlichen Gehörs. Gleiches gilt für die vom Übertragungselement 18 eingespeisten Vibrationen. Diese gelangen über die Knorpelmasse 22 und die Knochenstruktur 24 bis hin zum knöchernen Teil 40 der Schnecke 36. Dort wie auch auf dem Weg dahin können die Strahlung und die Vibrationen in Kombination ihre positiven Wirkungen entfalten und störende Ohrgeräusche lindem oder sogar im besten Fall einen chronischen Tinnitus beseitigen.

Die vorliegende Erfindung gibt damit ein Behandlungsgerät zur Reduzierung von Ohrgeräuschen, insbesondere zur Beseitigung von Tinnitus an. Dabei wird zumindest ein Abschnitt des sich von der Ohrmuschel bis zum Trommelfell erstreckenden Gehörganges bestrahlt und von einer Vibrationsquelle erzeugte Vibrationen aufgeprägt.

## Patentansprüche

1. Behandlungsgerät (1) zur Reduzierung von Ohrgeräuschen, insbesondere zur Beseitigung von Tinnitus, mittels Bestrahlung und Massage zumindest eines Abschnitts des sich von der Ohrmuschel bis zum Trommelfell erstreckenden Gehörganges, mit einem Gehäuse (6), in dem eine Energiequelle angeordnet ist, die mit einem Stromkreis und gegebenfalls einer Schaltung zur Regelung der Energiezufuhr mit einer Lichtquelle verbunden ist, **dadurch gekennzeichnet, dass** als Lichtquelle wenigstens eine Leuchtdiode vorgesehen ist, wobei das von der Leuchtdiode emittierte Licht von einem Lichtleiter (14) aufnehmbar und als Strahlung (17) dem zu bestrahlenden, den Gehörgang (2) begenzenden Gewebe zuführbar ist, und eine Vibrationsquelle im Gehäuse (6) angeordnet ist, die mit der Energiequelle über einen weiteren Stromkreis und gegebenenfalls einer Schaltung zur Regelung der Energiezufuhr verbunden ist, wobei die von der Vibrationsquelle erzeugten Vibrationen mit einem Übertragungselement (18) dem Gehörgang (2) in dessen vorderem, der Ohrmuschel zugewandten Eingangsbereich (20) aufprägbar sind.

2. Behandlungsgerät (1) nach Anspruch 1, **dadurch gekennzeichnet, dass** der in den Gehörgang (2) ragende Abschnitt (16) des Lichtwellenleiters (14) mit einem auswechselbaren Überzug (30) versehbar ist.

3. Behandlungsgerät (1) nach Anspruch 1 oder 2, **dadurch gekennzeichnet, dass** der Lichtwellenleiter (14) am lichtemmitierenden Ende gewölbt ausgebildet oder halbkugelartig gerundet ist, zur diffusen Verteilung der austretenden Strahlung, (17).

4. Behandlungsgerät. (1) nach einem der Ansprüche 1 bis 3, **dadurch gekennzeichnet, dass** wenigstens eine rote Leuchdiode mit hoher Leuchtstärke die Lichtquelle bildet.

5. Behandlungsgerät (1) nach einem der Ansprüche 1 bis 4, **dadurch gekennzeichnet, dass** die Vibrationsquelle einen Vibrationsmotor aufweist, der 2.000 bis 5.000 Schwingungen pro Minute, vorzugsweise maximal 4.500 Schwingungen pro Minute, besonders bevorzugt maximal 3.000 Schwingungen pro Minute, erzeugen kann.

6. Behandlungsgerät (1) nach einem der Ansprüche 1 bis 5, **dadurch gekennzeichnet, dass** das mit der Vibrationsquelle gekoppelte Übertragungselement (18) ringförmig ausgebildet ist.

7. Behandlungsgerät (1) nach einem der Ansprüche 1 bis 6, **dadurch gekennzeichnet, dass** der Lichtwellenleiter (14) durch das Übertragungselement (18) hindurch geführt ist.

8. Behandlungsgerät (1) nach einem der Ansprüche 1 bis 7, **dadurch gekennzeichnet, dass** die zum Gehörgang (2) gewandte Oberfläche des Übertragungselementes (18) hautfreundlich ausgebildet ist.

9. Behandlungsgerät (1) nach einem der Ansprüche 1 bis 8, **dadurch gekennzeichnet, dass** es einen Lautsprecher zur gezielten Angabe von Geräuschen oder Tonsignalen aufweist.

10. Behandlungsgerät (1) nach einem der Ansprüche 1 bis 9, **dadurch gekennzeichnet, dass** es einen Magneten (26) zur gezielten Abgabe von magnetischen Feldern aufweist.

11. Behandlungsgerät (1) nach einem der Ansprüche 1 bis 10, **dadurch gekennzeichnet, dass** das Gehäuse (6) wenigstens ein Bedienelement (8), wie beispielsweise einen Regelknopf, einen Taster, ein Touch-Screen, einen Multifunktionsschalter oder dergleichen aufweist, vermittels dem die Intensität und/oder die Dauer und/oder die Frequenz der Strahlungen und/oder Vibrationen und/oder anderer Funktionen ein- bzw. ausschaltbar und/oder regelbar einstellbar sind, vorzugweise vorprogrammierte Steuerprogramme abrufbar sind.

## Claims

1. Apparatus (1) for reducing earnoise, in particular for removing tinnitus, by means of radiation and massage of at least a region of the auditory canal (2) extending from the auricle to the eardrum, having a housing (6) comprising an energy source arranged therein and connected to an electric circuit and eventually to a circuit for controlling the energy input with a light source, **characterized in that** as said light source at least a light emitting diode is provided, wherein the light emitted by said light emitting diode is inputted to a light guide (14) and is supplied as radiation (17) to the irradiated tissue of the auditory canal (2), and wherein a vibration source is arranged in the housing (6), which is connected to said energy source and via another electric circuit and eventually via a circuit for controlling the energy source, whereby the vibrations produced by said vibration source are input by means of a transmission element (18) to said auditory canal (2) in its front entrance area facing said auricle.

2. Apparatus (1) according to claim 1,
**characterized in that** the part of the light guide (14) extending into the auditory canal (2) is covered with an exchangeable cover (30).

3. Apparatus (1) according to claim 1 or 2,
**characterized in that** said light guide (14) at its lightemitting end is curved or rounded hemispherical, in order to diffusly distributing the emitted radiation (17).

4. Apparatus (1) according to one of claims 1 through 3,
**characterized in that** said light source is provided by at least one red light emmitting diode having a high luminosity.

5. Apparatus (1) according to one of claims 1 through 4,
**characterized in that** said vibration source comprises a vibration motor which generates oscillations from 2.000 to 5.000 per minute, preferable a maximum of 4.500 oscillations per minute, and especially preferable maximum of 3.000 oscillations per minute.

6. Apparatus (1) according to one of claims 1 through 5,
**characterized in that** said transmission element (18) coupled to said vibration source is formed annular .

7. Apparatus (1) according to one of claims 1 through 6,
**characterized in that** said light guide (14) is guided through said transmission element (18).

8. Apparatus (1) according to one of claims 1 through 7,
**characterized in that** the surface of the transmission element (18) faced to the auditory canal (2) is formed pleasant to skin.

9. Apparatus (1) according to one of claims 1 through 8,
**characterized in that** it comprises a loudspeaker to emit well directed noise or sound signals.

10. Apparatus (1) according to one of claims 1 through 9,
**characterized in that** it comprises a magnet (26) for a well directed emission of magnetic fields.

11. Apparatus (1) according to one of claims 1 through 10,
**characterized in that** said housing (6) comprises at least one control element (8) like a regulation button, a pushbutton, a touch screen, a multifunction button or simlar, by which the intensity and/or the duration and/or the frequency of said radiation and/or said vibration and/or other functions that could be turned on or off and/or could be adjustable, in particular by a preprogrammed control program.

## Revendications

1. Dispositif (1) de traitement du bourdonnement d'oreille, en particulaire de suppresser du tinnitus, par radiation et massage au moins une region d'une canale d'oreille (2) que s'entends au pavillion d'oreille du tympan, et que a une boîte (6) que comprends une source d'énergie connectée avec une circuit electrique et éventuelle avec une circuit de controllé l'energie entré avec une source lumineuse, **caractérisé en ce que** la source lumineuse comprends au moins un diode lumineuse, et la radiation emitté par le diode lumineuse est entrée d'un conduit du lumière (14) et est ajouté du radiation (17) à la tissue radié du canale d'oreille (2), et une source de vibration est arrangé de la boîte (6), que est connecté avec la source d'énergie et une autre circuit electrique et éventuelle à une circuit de controllé la source d'énergie, et que les vibrations produit par la source de vibration sont entrés par un élément du transmission au canale d'oreille (2).

2. Dispositif (1) selon la revendication 1,
**caractérisé en ce que** le part du conduit du lumière (14) que s'entends au canale d'oreille (2) est couvert par une couverture.

3. Dispositif (1) selon la revendication 1 ou 2,
**caractérisé en ce que** le conduit du lumière (14) est incurvé ou globale pour distributé diffusement le radiation (17).

4. Dispositif (1) selon l'une des revendications 1 à 3,
**caractérisé en ce que** au moins un rouge diode lumineuse avec une grande luminosité configures la source lumineuse.

5. Dispositif (1) selon l'une des revendications 1 à 4,
**caractérisé en ce que** la source de vibration comprends un moteur du vibration que produit oscillations de 2.000 à 5.000 par minute, preferable à une maximum de 4.500 oscillations, et en particulaire preferable à une maximum de 3.000 oscillations par minute.

6. Dispositif (1) selon l'une des revendications 1 à 5,
**caractérisé en ce que** l'élément du transmission (18) couplé de la source de vibration est formé annulaire.

7. Dispositif (1) selon l'une des revendications 1 à 6,
**caractérisé en ce que** le conduit du lumière (14) est conduit par l'élément du transmission (18).

8. Dispositif(1) selon l'une des revendications 1 à 7,
**caractérisé en ce que** le surface d'élément du transmission est agréable pour la peau.

9. Dispositif (1) selon l'une des revendications 1 à 8,
**caractérisé en ce que** il comprends un haut-parleur pour emité directement des signaux des sons.

10. Dispositif (1) selon l'une des revendications 1 à 9,
**caractérisé en ce que** il comprends un aimant (26) pour directement generé un champ de magnetique.

11. Dispositif (1) selon l'une des revendications 1 à 10,
**caractérisé en ce que** la boîte (6) comprends au moins un élément du controlé (8), par example une bouton du reglage, une bouton de presser, une screen de touché, une bouton du multifunction, au moins lequel l'intensité et/ou la duration et/ou la frequence du radiation et/ou la vibration et/ou autre functions sont adjustable.
